# FASCICULE DE BREVET EUROPEEN

(11) **EP 3 203 934 B1**
(45) Date de publication et mention de la délivrance du brevet: **06.09.2023**
(21) Numéro de dépôt: 15801894.5
(22) Date de dépôt: 12.10.2015
(51) Int. Cl.: A61F 2/28, A61F 2/30, A61B 17/86

(54) **BLOC DE SYNTHÈSE DESTINÉ A COMBLER UN DÉFAUT OSSEUX ET SON PROCÉDÉ DE FABRICATION**
SYNTHETISCHER BLOCK ZUM AUSFÜLLEN EINES KNOCHENDEFEKTS UND VERFAHREN ZUR HERSTELLUNG DAVON
SYNTHETIC BLOCK INTENDED FOR FILLING IN A BONE DEFECT AND METHOD FOR MANUFACTURING SAME

(30) Priorité: 10.10.2014 FR 1459765
(43) Date de publication de la demande: 16.08.2017
(73) Titulaire: S.A.S 3DCeram-Sinto, 87280 Limoges (FR)
(72) Inventeur: GAIGNON, Richard, 91770 Saint-Vrain (FR); CHAPUT, Christophe, 87410 Le Palais sur Vienne (FR)
(74) Mandataire: Cabinet Chaillot
(86) Numéro de dépôt international: PCT/FR2015/052748
(87) Numéro de publication internationale: WO 2016/055752

(56) Documents cités:
- EP-A2- 1 772 108
- WO-A1-2009/004070
- WO-A1-2013/181375
- WO-A2-2009/129000
- US-A1- 2005 085 922

## Description

La présente invention concerne un procédé de fabrication d'un bloc de synthèse destiné à combler un défaut osseux à la surface d'un os.

En particulier, le défaut osseux traité conformément à l'invention est un défaut osseux apparaissant dans l'os d'une mandibule ou d'un maxillaire. Toutefois, l'invention n'est pas limitée à de tels défauts osseux.

Le volume osseux des maxillaires est une donnée essentielle à la mise en place d'implants, à savoir de racines artificielles pour le remplacement de dents absentes.

Actuellement quand le volume osseux est insuffisant, il est possible :
- pour des volumes faibles, d'utiliser des produits de comblement sous forme de granules ou de pâte d'origine synthétique (phosphate tricalcique bêta, hydroxyapatite), humaine ou animale (bovine, porcine, équine) : une telle application constitue la Régénération Osseuse Guidée (ROG) ;
- pour des volumes importants, il est nécessaire de faire des greffes d'apposition avec des blocs d'origine humaine (os de banque), animale ou synthétique, ou avec des blocs d'os du patient opéré, autogreffé par prélèvement symphysaire, ramique ou pariétal.

Ces techniques connues présentent des difficultés car les blocs précités posent des problèmes de forme et de quantité. En effet, ils sont de taille standard et nécessitent donc d'être retaillés en peropératoire (pendant l'opération) pour les adapter le mieux possible au défaut osseux.

Il en découle :
- des problèmes de stabilité car les blocs et le défaut osseux ne sont jamais parfaitement jointifs ;
- des risques de fracture par bascule du bloc au moment de l'ostéosynthèse (fixation par vis) ;
- des problèmes d'ostéointégration (colonisation du bloc par des cellules osseuses et formation de néovaisseaux) et donc un rejet du bloc ;
- des problèmes de bordures trop tranchantes avec un risque de lésion des tissus mous (tissus de recouvrement, gencive, épithélium, conjonctif) donc une mauvaise cicatrisation tissulaire pourtant indispensable ; les sutures des tissus mous doivent permettre une étanchéité complète et sans tension pour espérer avoir une ostéointégration du bloc ;
- pour les autogreffes s'ajoute le problème d'un deuxième site opératoire avec les suites qui en découlent (prélèvement au ramus, à la symphyse mentonnière ou en anesthésie générale à l'os pariétal ou à la hanche) ;
- pour les autogreffes se pose également le problème de la quantité qu'il est possible de prélever.

La présente invention a pour but de remédier à ces inconvénients.

WO 2009/00470 divulgue un implant chirurgical qui comprend une partie de cœur poreuse faite de matière biocompatible et une enveloppe dense disposée pour configurer une interface entre la partie de cœur et les tissus mous et pour empêcher la croissance des tissus mous dans la partie de cœur.

WO 2013/1811375 décrit un dispositif de réparation des tissus ou échafaudage ayant une structure de croissance osseuse poreuse contenant des jambes interconnectées entourées par une enveloppe microporeuse.

US 2005/085922 décrit une pièce de remplissage de vide osseux en forme de coins, de tranches ou de pièces à symétrie axiale, en particulier pour réduire ou arrêter l'écoulement de sang à partir d'un os coupé.

A cet effet, selon l'invention, il est proposé de réaliser par la technique par procédés additifs-pouvant aussi être appelée stéréolithographie ou impression 3D - des blocs de synthèse en matériau céramique parfaitement adaptés aux défauts osseux des patients, ceci permettant de répondre aux différentes difficultés rencontrées avec les techniques actuelles.

En effet, étant réalisé par impression 3D (stéréolithographie) à partir des données scanner du patient (fichiers STL), le bloc est parfaitement adapté au défaut :
- on supprime les états de retouche peropératoire, difficiles et non sans risque pour le bloc ;
- on supprime le problème d'adaptation du bloc au défaut osseux ;
- on obtient un joint de meilleure qualité, donc un contact étroit entre le bloc et les cellules osseuses sanguines ;
- on réalise le corps du bloc avec une « porosité » suffisante permettant une colonisation des cellules osseuses et une néovascularisation (fabrication de nouveaux vaisseaux sanguins) ;
- on place des puits à des endroits précis pour le passage d'une de plusieurs vis de stabilisation (ostéosynthèse) ; il faut notamment un renforcement du bloc au niveau de l'appui des têtes de vis et du chanfrein et le diamètre de ces trous est adapté au diamètre des vis pour éviter de créer une tension et des fractures du bloc au moment du serrage de celle-ci ;
- la composition du bloc en matériau céramique est indispensable pour avoir une résorption totale partielle programmée du bloc et son remplacement total ou partiel par l'os du patient néoformé ; en effet l'opérateur doit réintervenir sur le site opéré après la fin de cicatrisation osseuse complète pour venir mettre en place le ou les implants pour lequel l'augmentation du volume osseux de la mandibule ou du maxillaire était nécessaire ;
- les problèmes rencontrés avec les autogreffes (deuxième site opératoire et quantité prélevée) sont évidemment totalement écartés.

La présente invention a donc d'abord pour objet un procédé de fabrication d'un bloc de synthèse destiné à combler un défaut osseux en surface d'un os, suivant l'objet de la revendication 1.

Le réseau tridimensionnel de canaux est un réseau ordonné, par conséquent différent d'un ensemble de pores interconnectés ouverts, tel que celui décrit dans WO 2009/004070 A1. Le réseau ordonné est obtenu par stéréolithographie ou impression 3D ou technique des procédés additifs, ce qui permet de maîtriser la structure du réseau, alors que l'ensemble de pores interconnectés ouverts est aléatoire, donc non maîtrisable. On pourra ainsi fabriquer la pièce en fonction de la revascularisation souhaitée. De plus, la pièce pourra comporter des réseaux de canaux de densité différente (nombre de canaux par cm² ou cm³).

Le matériau céramique est avantageusement un matériau céramique au moins en partie résorbable. Le matériau céramique peut également être un matériau céramique non résorbable.

Le matériau céramique est notamment choisi parmi le phosphate tricalcique bêta (β-TCP), l'hydroxyapatite et leurs mélanges en toutes proportions, étant en particulier composé, pour 100% en poids, de 40 à 100 % en poids d'hydroxyapatite et de 60 à 0 % en poids de β-TCP. Un mélange courant consiste en 60% en poids d'hydroxyapatite et 40% en poids de β-TCP. Le β-TCP est résorbable alors que l'hydroxyapatite ne l'est pas.

Le réseau tridimensionnel de canaux peut être de n'importe quelle forme dans la mesure où il permet la revascularisation par la pénétration des fluides et cellules nécessaires à cette revascularisation ; on peut citer en particulier les réseaux à mailles cubiques, les canaux s'étendant alors selon chacune des arêtes du réseau à mailles cubiques.

La pièce en céramique peut porter extérieurement, d'un seul tenant avec elle, au moins un oeillet de stabilisation destiné à venir s'appliquer contre la surface de l'os à restaurer, extérieurement audit défaut osseux, ledit oeillet de stabilisation ne comportant pas de canaux de revascularisation et étant percé d'au moins un trou pour le passage d'au moins une vis de stabilisation, et/ou ladite pièce en céramique peut être percée d'au moins un trou traversant, allant de la surface destinée à venir en contact avec l'os délimitant ledit défaut osseux à la surface libre si l'on considère la position de la pièce en place dans le défaut osseux, en vue du passage d'au moins une vis de stabilisation, ladite pièce ne comportant pas de canaux de revascularisation dans les régions entourant ledit trou au moins dans la partie voisine de ladite surface libre.

Avantageusement, la pièce ne comporte pas de canaux de revascularisation dans la région de sa surface libre si l'on considère sa position en place dans le défaut osseux.

Les canaux formant le système de revascularisation peuvent être de toute section, par exemple circulaire, carrée, triangulaire, en losange, avec des formes présentant le plus grand nombre d'angles (en forme de croix par exemple). En particulier, les canaux formant le système de revascularisation peuvent avoir une section carrée dont le côté est de 250 à 600 µm avec une tolérance de 200 µm.

De façon générale, les canaux de revascularisation peuvent être de section variable, rectilignes ou non, débouchants ou non à l'opposé de la surface de la pièce destinée à venir en contact avec le défaut osseux car la structure de ces canaux est maîtrisée.

Les canaux formant le système de revascularisation ont avantageusement une section supérieure dans la région de la pièce destinée à être en contact avec l'os délimitant le défaut osseux, étant notamment des canaux de section carrée de 400 à 600 µm de côté avec une tolérance de 200 µm, les canaux de coeur de la pièce étant des canaux de section carrée de côté plus petit ; en variante, la densité des canaux formant le système de revascularisation peut être supérieure dans la ou les régions de la pièce destinées à être en contact avec le défaut osseux.

Le matériau céramique composant la pièce a notamment une microporosité intergranulaire, mesurée par porosimétrie au mercure, de 5 à 30 % en volume, les micropores ayant une dimension de 0,1 à 10 µm. Cette microporosité est propre au matériau céramique fabriqué.

Il est intéressant que :
- le coeur de la pièce ait une structure se rapprochant au maximum de l'os trabéculaire « poreux » et tendre ;
- la périphérie externe de la pièce (considérée dans sa position mise en place dans le défaut osseux) ait une structure se rapprochant au maximum des caractéristiques de l'os cortical : dense et rigide ; et
- la partie de la pièce destinée à venir au contact de l'os du patient soit très « poreuse » pour permettre une revascularisation la plus rapide possible.

On décrit ci-dessus par « poreux » ou « poreuse » la présence du réseau de canaux tel qu'il a été défini ci-dessus. En effet la périphérie externe de la pièce a la même structure que le reste de la pièce, avec la microporosité inhérente au procédé de fabrication.

Le procédé de fabrication de la pièce en matière céramique telle que définie ci-dessus comprend généralement les étapes consistant à :
- former, sur un support rigide ou sur une pièce en cours de fabrication, une première couche d'une composition photodurcissable comprenant au moins une matière céramique et un monomère photodurcissable ;
- faire durcir la première couche de la composition photodurcissable, par irradiation selon le motif défini pour ladite couche, formant un premier étage;
- former, sur le premier étage, une seconde couche de la composition photodurcissable ;
- faire durcir la seconde couche de la composition photodurcissable, par irradiation selon le motif défini pour ladite couche, formant un deuxième étage ;
- optionnellement répéter lesdites étapes pour obtenir une pièce à l'état cru ;
- nettoyer la pièce à l'état cru pour enlever la composition non durcie ;
- optionnellement délianter la pièce à l'état cru nettoyée ;
- fritter la pièce à l'état cru nettoyée et optionnellement déliantée, pour obtenir la pièce finie.

Dans un mode de réalisation particulier du procédé, on fabrique la pièce par stéréolithographie en voie liquide, la composition photodurcissable étant liquide et le support rigide étant une plate-forme immergée dans un bain de composition photodurcissable, et l'on forme chacune des couches de la composition photodurcissable par abaissement de la plateforme dans le bain de composition photodurcissable de telle sorte que l'étage supérieur de la pièce en cours de fabrication soit abaissé sous la surface libre de la composition photodurcissable, et l'on fait durcir chacune des couches de composition photodurcissable par balayage laser de ladite surface libre selon le motif défini pour ladite couche.

Dans un autre mode de réalisation particulier du procédé, on fabrique la structure poreuse par stéréolithographie en voie pâteuse, la composition photodurcissable étant pâteuse et on apporte la composition photodurcissable sur l'étage supérieur de la pièce en cours de fabrication et on étale la composition photodurcissable pour former chacune des couches de composition photodurcissable, et l'on fait durcir chacune des couches de composition photodurcissable par balayage laser de ladite couche selon le motif défini pour ladite couche.

Pour mieux illustrer l'objet de la présente invention, on va en décrire ci-après, à titre indicatif et non limitatif, plusieurs modes de réalisation avec référence au dessin annexé.

Sur ce dessin :
- la Figure 1 est une vue schématique en perspective de la mandibule saine d'un homme adulte ;
- la Figure 2 est une vue du corps de la mandibule de la Figure 1 comportant un défaut osseux ;
- la Figure 3 est une vue schématique de profil d'un bloc destiné à combler ce défaut osseux, bloc conforme à un premier mode de réalisation de l'invention ;
- la Figure 4 est une vue schématique de dessus du bloc de la Figure 3 ;
- la Figure 5 est une vue correspondant à la Figure 2 après mise en place et stabilisation du bloc de synthèse selon le premier mode de réalisation de l'invention ;
- la Figure 6 est une vue correspondant à la Figure 5 après résorption du matériau du bloc de synthèse et remplacement par l'os du patient et mise en place de racines artificielles ;
- les Figures 7 à 10 sont des vues frontales d'un maxillaire respectivement l'état sain ; après perte du bloc incisif ; après mise en place d'un bloc de synthèse selon le premier mode de réalisation de la présente invention ; et après cicatrisation osseuse complète et mise en place des implants dentaires ;
- les Figures 11 et 12 sont des vues correspondant aux Figures respectivement 3 et 4, montrant un bloc de synthèse réalisé conformément à un second mode de réalisation de la présente invention, les vis de fixation utilisées avec ce second mode de réalisation ayant été représentées sur ces Figures 11 et 12 ;
- la Figure 13 est une vue en coupe transversale du bloc de synthèse sur le premier ou second mode de réalisation en vue d'en décrire une structure possible ;
- la Figure 14 est une vue de dessus d'une partie de mandibule comportant un défaut osseux comblé par un bloc de synthèse conforme au second mode de réalisation de l'invention ; et
- la Figure 15 représente, à plus grande échelle, une vue en coupe longitudinale du bloc de synthèse de la Figure 14, en vue d'en décrire la structure.

Sur les vues schématiques anatomiques des dessins, pour des raisons de clarté, on a fait abstraction des tissus mous : gencive, muscles, joues, etc.... et du système vasculaire, seuls les tissus durs : os et dents apparaissant.

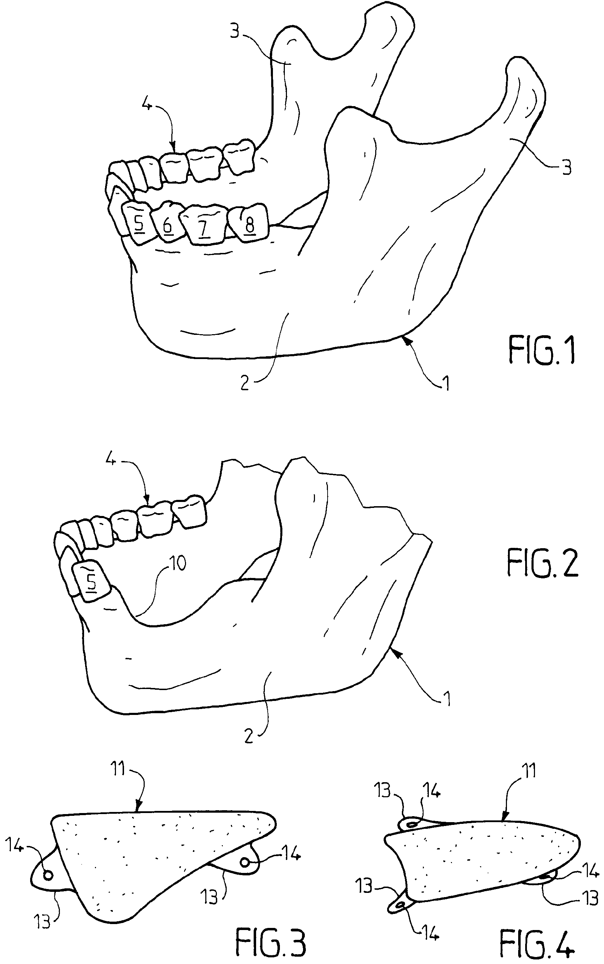

Si l'on se réfère à la Figure 1, on peut voir que l'on a représenté la mandibule 1 d'un homme adulte, avec son corps 2 et ses deux branches 3. Le corps 2 porte les dents 4 de l'arcade dentaire inférieure, celles-ci étant enfoncées dans les alvéoles creusées dans le bord alvéolaire spongieux du corps de mandibule. Sur le dessin, les dents n'ont pas été représentées de manière exacte, l'objectif de l'invention étant de présenter un bloc de synthèse destiné à combler un défaut osseux et sa mise en place dans celui-ci. Les dents concernées ici ont été numérotées par leur position par rapport au milieu de la mandibule, à savoir : 5 : deuxième prémolaire ; 6 : première molaire ; 7 : deuxième molaire ; 8 : troisième molaire ou dent de sagesse

Sur la Figure 2, on a représenté le corps de mandibule après perte des dents 6, 7 et 8, et de l'os alvéolaire associé.

Le défaut osseux 10 qui s'est ainsi formé présente la forme d'une auge qui s'étend d'une paroi latérale à l'autre du corps de mandibule.

La pièce en matière céramique 11 destinée à venir combler ce défaut 10 est représentée sur les Figures 3 et 4.

Elle comporte un corps 12 qui présente une forme lui permettant d'épouser parfaitement le défaut 10, et qui porte extérieurement trois oeillets 13 dans l'exemple représenté, à savoir deux oeillets d'un côté et un de l'autre.

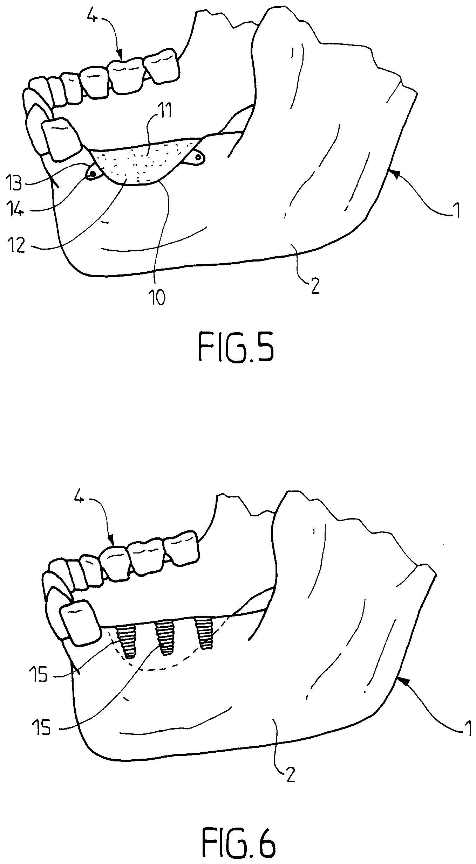

Les oeillets 13 sont destinés à venir s'appliquer contre les parois latérales respectives de la mandibule comme on peut le voir sur la Figure 5. Ils comportent chacun un trou 14 pour le passage d'une vis d'ostéosynthèse permettant de stabiliser la pièce 11 une fois en place dans le défaut 10. L'axe des trous 14 est orienté pour obtenir l'orientation voulue des vis d'ostéosynthèse en vue d'un accrochage des oeillets 13 pour une stabilisation parfaite de la pièce 11. De même, les oeillets 13 sont positionnés sur le corps 12 de la pièce 11 pour assurer une telle stabilisation.

Sur la Figure 6, on a représenté la mandibule 1 après résorption du matériau céramique de la pièce 11 et remplacement par l'os du patient. Après cicatrisation, la mise en place d'implants dentaires 15 est rendue possible.

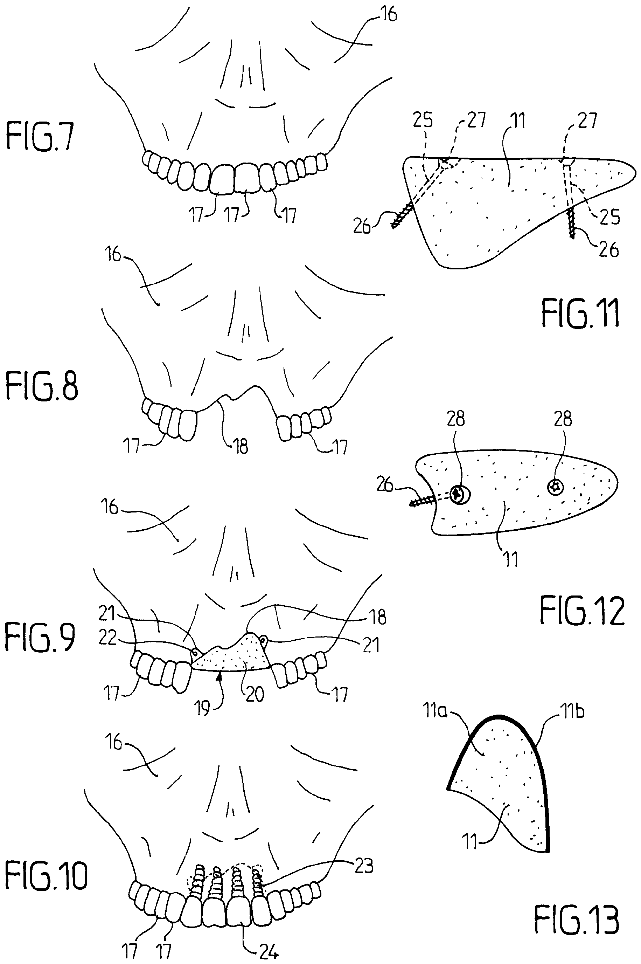

La structure de la pièce 11 sera décrite ci-après avec référence à la Figure 13.

Les Figures 7 à 10 correspondent aux vues respectivement 1, 2, 5 et 6 pour un maxillaire 16 :
- la Figure 7 représente une vue frontale du maxillaire 16 portant les dents 17 de l'arcade dentaire supérieure ;
- la Figure 8 représente la vue frontale correspondante avec perte des quatre incisives et perte osseuse, créant le défaut osseux 18 ;
- la Figure 9 montre la pièce 19 en matériau céramique selon l'invention dont le corps 20 est venu combler le défaut osseux et les oeillets 22 ont été appliqués contre la paroi avant du maxillaire, permettant le passage de vis d'ostéosynthèse à travers les trous correspondants 22 ; et
- la Figure 10 montre la vue frontale du maxillaire après cicatrisation osseuse complète et mise en place des quatre implants dentaires sur lesquels ont été posées les nouvelles dents 24.

Les Figures 11 et 12 sont des vues correspondant aux Figures respectivement 3 et 4, mais avec un autre mode de réalisation des moyens de stabilisation de la pièce 11.

Dans ce mode de réalisation, des trous traversants ou perçages 25 sont forés à travers la pièce 11 (deux perçages 25 dans l'exemple représenté) pour le passage de la vis d'ostéosynthèse 26 (représentée sur les Figures 11 et 12) devant pénétrer dans l'os du patient délimité par le défaut osseux 10. A l'opposé, chaque perçage 25 s'évase selon une partie chanfreinée 27 pour le logement de la tête de vis 28 correspondante. Le positionnement et l'orientation des axes des vis 26 sont choisis pour assurer une bonne stabilisation de la pièce 11 en vue d'une revascularisation réussie.

Si l'on se réfère à la Figure 11, on peut voir que la pièce 11 peut comporter deux types de « porosité », à savoir :
- une partie principale 11a dans laquelle le réseau tridimensionnel de canaux de revascularisation est formé de canaux de section carrée par exemple de 250 à 600 µm +/- 200 µm de côté ; et
- une partie de surface 11b qui ne présente pas de réseau de revascularisation, donc sans canaux (avec seulement la microporosité) pour une meilleure résistance.

Comme indiqué plus haut, la partie 11a pourrait présenter un réseau plus dense ou avec des sections de canaux plus grandes dans sa région de contact avec l'os du patient en vue d'une accélération de la revascularisation.

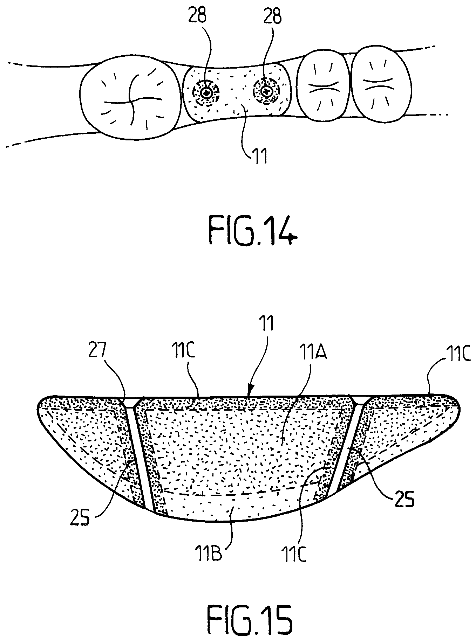

Les Figures 14 et 15 montrent une pièce 11 conforme au second mode de réalisation qui comporte trois régions différentes quant à sa « porosité » :
- une partie de coeur 11A dans laquelle le réseau tridimensionnel de canaux de revascularisation est formé de canaux de section carrée par exemple de 250-350 µm +/- 200 µm de côté ;
- une partie 11B destinée à venir en contact avec l'os du patient, dans laquelle le réseau tridimensionnel de canaux de vascularisation est plus dense ou est formé de canaux de section carrée de section plus grande que les canaux de la partie 11A, par exemple de 400 à 600 µm +/-200 µm de côté ; et
- une partie 11C de surface et entourant les perçages 25 qui ne présente pas de réseau de revascularisation, donc sans canaux (avec seulement la microporosité) pour une meilleure résistance.

La structure selon l'invention peut être fabriquée selon tout procédé de fabrication couche par couche de la matière céramique.

A titre d'exemple de tels procédés, on peut citer le prototypage rapide, et notamment la stéréolithographie. Ce procédé est connu de l'homme du métier, et, pour en avoir une description détaillée, on se reportera aux brevets US5496682 ou EP1472081.

Brièvement, en stéréolithographie par voie pâteuse, on prépare une pâte ayant par exemple la composition suivante (en % de la masse totale) :

| | |
|---|---|
| céramique | 80 |
| liant photopolymérisable | 11,51 |
| photoinitiateur | 0,09 |
| dispersant | 1,1 |
| plastifiant | 7,3 |

La céramique est ici de l'hydroxyapatite ou la β-TCP ou un mélange des deux. Le liant photopolymérisable peut être une résine acrylate, telle que le diméthacrylate de bisphénol A di-éthoxylé ou le diacrylate de 1, 6 hexanediol. Le photoinitiateur sera choisi parmi les photoinitiateurs couramment utilisés dans la polymérisation des acrylates. On citera notamment le 2,2'-diméthoxy-2-phénylacétophénone et le 2-hydroxy-2-méthyl-1-phényl-propane-1-one. Le dispersant est avantageusement un ester phosphorique. Comme plastifiant on peut choisir un ou plusieurs agents du groupe constitué par la famille des glycols (ex : le polyéthylène glycol), la famille des phtalates (ex : le dibutylphtalate), le glycérol.

Dans un appareil de stéréolithographie par voie pâteuse, on commence par étaler la pâte sur une plateforme pour former une première couche d'épaisseur uniforme. On irradie cette première couche par balayage laser selon le motif défini pour la couche. La première couche de pâte est durcie par photopolymérisation de la pâte, sauf dans les zones correspondant aux canaux, qui ne sont pas irradiées par le laser. On étale ensuite une seconde couche de pâte sur la première couche durcie. On irradie cette seconde couche par balayage laser selon le motif défini pour la couche. La seconde couche de pâte est donc durcie, par photopolymérisation de la pâte, sauf dans les zones correspondant aux canaux. On répète ces opérations pour former les étages suivants.

Chacune des couches formées a une épaisseur de 25 à 100 µm, notamment de 50 µm ; il va de soi que le nombre de couches dépend de la pièce fabriquée.

Après photopolymérisation de la dernière couche, on nettoie la pièce à l'état cru ainsi formée pour éliminer la composition non polymérisée. On soumet la pièce crue nettoyée à un traitement thermique (déliantage) puis à un frittage.

Il est bien entendu que les modes de réalisations qui ont été décrit ci-dessus ont été donnés à titre indicatif et non limitatif et que des modifications peuvent être apportées sans que l'on s'écarte pour autant du cadre de la présente invention.

## Revendications

1. Procédé de fabrication d'un bloc de synthèse destiné à combler un défaut osseux (10 ; 18) en surface d'un os, en particulier un défaut osseux apparaissant dans l'os d'une mandibule ou d'un maxillaire, **caractérisé par le fait qu'**il comporte les étapes suivantes :
- on fait l'acquisition d'une image tridimensionnelle de l'os d'un patient présentant le défaut osseux (10 ; 18) que l'on veut combler ;
- on construit, par conception assistée par ordinateur, un modèle informatique du bloc de synthèse dont la forme correspond au défaut osseux (10 ; 18), qui comporte les canaux de revascularisation et dont les dimensions sont légèrement plus grandes que ledit défaut osseux (10 ; 18) pour tenir compte du retrait de la céramique au cours de la fabrication du bloc de synthèse ;
- on modifie ce modèle informatique de bloc de synthèse, par conception assistée par ordinateur, pour que puisse être assurée la stabilisation dudit bloc de synthèse dans ledit défaut osseux (10 ; 18) ; et
- on fabrique le bloc de synthèse recherché, en matériau céramique, par stéréolithographie, un réseau tridimensionnel ordonné de canaux communiquant entre eux étant formé au moins en partie dans ledit bloc (11) pour laisser passer les fluides et cellules permettant une revascularisation en vue de la croissance cellulaire une fois ledit bloc (11) en place dans le défaut osseux (10 ; 18), lesdits canaux débouchant sur chaque surface du défaut osseux (10 ; 18) en contact avec ledit bloc (11) une fois mis en place dans le défaut osseux (10 ; 18).

2. Procédé selon la revendication 1, **caractérisé par le fait que** le matériau céramique est un matériau céramique au moins en partie résorbable, ou un matériau céramique non résorbable.

3. Procédé selon l'une des revendications 1 et 2, **caractérisé par le fait que** le matériau céramique est choisi parmi le phosphate tricalcique bêta (β-TCP), l'hydroxyapatite et leurs mélanges en toutes proportions, étant en particulier composé, pour 100% en poids, de 40 % à 100 % en poids d'hydroxyapatite et de 60 % à 0 % en poids de β-TCP.

4. Procédé selon l'une des revendications 1 à 3, **caractérisé par le fait que** le bloc (11) en céramique porte extérieurement, d'un seul tenant avec lui, au moins un œillet de stabilisation (13) destiné à venir s'appliquer contre la surface de l'os à restaurer, extérieurement audit défaut osseux (10 ; 18), ledit œillet de stabilisation (13) ne comportant pas de canaux de revascularisation et étant percé d'au moins un trou (14) pour le passage d'au moins une vis de stabilisation, et/ou que ledit bloc (11) en céramique est percé d'au moins un trou traversant (25), allant de la surface destinée à venir en contact avec l'os délimitant ledit défaut osseux (10 ; 18) à la surface libre si l'on considère la position du bloc (11) en place dans le défaut osseux (10 ; 18), en vue du passage d'au moins une vis de stabilisation (26), ledit bloc (11) ne comportant pas de canaux de revascularisation dans les régions entourant ledit trou (25) au moins dans la partie voisine de ladite surface libre.

5. Procédé selon l'une des revendications 1 à 4, **caractérisé par le fait que** le bloc (11) ne comporte pas de canaux de revascularisation dans la région de sa surface libre si l'on considère la position du bloc (11) en place dans le défaut osseux (10 ; 18).

6. Procédé selon l'une des revendications 1 à 5, **caractérisé par le fait que** les canaux de revascularisation sont de section variable, rectilignes ou non, débouchants ou non à l'opposé de la surface du bloc (11) destinée à venir en contact avec le défaut osseux (10 ; 18).

7. Procédé selon l'une des revendications 1 à 6, **caractérisé par le fait que** les canaux formant le système de revascularisation ont une section carrée dont le côté est de 250 à 600 µm avec une tolérance de 200 µm.

8. Procédé selon l'une des revendications 1 à 7, **caractérisé par le fait que** les canaux formant le système de revascularisation ont une section supérieure dans la région du bloc (11) destinée à être en contact avec l'os délimitant le défaut osseux (10 ; 18), étant notamment des canaux de section carrée de 400 à 600 um de côté avec une tolérance de 200µm, les canaux du coeur de la pièce étant des canaux de section carrée de côté plus petit, ou la densité des canaux formant le système de revascularisation est supérieure dans la ou les régions du bloc (11) destinées à être en contact avec le défaut osseux (10 ; 18).

9. Procédé selon l'une des revendications 1 à 8, **caractérisé par le fait que** le matériau céramique composant le bloc (11) a une microporosité intergranulaire de 5 à 30 % en volume, les micropores ayant une dimension de 0,1 à 10 µm.

10. Procédé selon l'une des revendications 1 à 9, **caractérisé par le fait que** l'on fabrique le bloc de synthèse recherché par les étapes consistant à :
- former, sur un support rigide ou sur une pièce en cours de fabrication, une première couche d'une composition photodurcissable comprenant au moins une matière céramique et un monomère photodurcissable ;
- faire durcir la première couche de la composition photodurcissable, par irradiation selon le motif défini pour ladite couche, formant un premier étage ;
- former, sur le premier étage, une seconde couche de la composition photodurcissable ;
- faire durcir la seconde couche de la composition photodurcissable, par irradiation selon le motif défini pour ladite couche, formant un deuxième étage ;
- optionnellement répéter lesdites étapes pour obtenir une pièce à l'état cru ;
- nettoyer la pièce à l'état cru pour enlever la composition non durcie ;
- optionnellement délianter la pièce à l'état cru nettoyée ;
- fritter la pièce à l'état cru nettoyée et optionnellement déliantée, pour obtenir la pièce finie.

## Patentansprüche

1. Verfahren zur Herstellung eines synthetischen Blocks, der dazu bestimmt ist, einen Knochendefekt (10; 18) an der Oberfläche eines Knochens zu füllen, insbesondere einen Knochendefekt, der im Knochen eines Unterkiefers oder eines Oberkiefers auftritt, **dadurch gekennzeichnet, dass** es die folgenden Schritte aufweist:
- Erfassen eines dreidimensionalen Bildes des Knochens eines Patienten, der den Knochendefekt (10; 18) aufweist, den man füllen will;
- Konstruieren, durch computergestütztes Design, eines Computermodells des synthetischen Blocks, dessen Form dem Knochendefekt (10; 18) entspricht, das die Revaskularisationskanäle aufweist und dessen Abmessungen etwas größer sind als der Knochendefekt (10; 18), um das Entfernen der Keramik während der Herstellung des synthetischen Blocks zu berücksichtigen;
- Verändern dieses Computermodells des synthetischen Blocks durch computergestütztes Design, um die Stabilisierung des synthetischen Blocks im Knochendefekt (10; 18) sichern zu können; und
- Herstellen des angestrebten synthetischen Blocks aus Keramikmaterial durch Stereolithographie, wobei ein geordnetes dreidimensionales Netzwerk von miteinander kommunizierenden Kanälen mindestens zum Teil in dem Block (11) gebildet wird, um die Fluide und Zellen passieren zu lassen, die eine Revaskularisation im Hinblick auf das Zellwachstum gestatten, sobald der Block (11) im Knochendefekt (10; 18) platziert ist, wobei die Kanäle auf jeder Oberfläche des Knochendefekts (10; 18) im Kontakt mit dem Block (11) ausmünden, sobald dieser im Knochendefekt (10; 18) platziert ist.

2. Verfahren nach Anspruch 1, **dadurch gekennzeichnet, dass** das Keramikmaterial ein mindestens zum Teil resorbierbares Keramikmaterial oder ein nicht resorbierbares Keramikmaterial ist.

3. Verfahren nach einem der Ansprüche 1 und 2, **dadurch gekennzeichnet, dass** das Keramikmaterial aus dem beta-Tricalciumphosphat (β-TCP), dem Hydroxyapatit und deren Gemischen in allen Verhältnissen ausgewählt ist, wobei es insbesondere für 100 Gew.-% aus 40 bis 100 Gew.-% Hydroxyapatit und aus 60 bis 0 Gew.-% β-TCP zusammengesetzt ist.

4. Verfahren nach einem der Ansprüche 1 bis 3, **dadurch gekennzeichnet, dass** der Keramikblock (11) außen einteilig mit ihm mindestens eine Stabilisierungsöse (13) trägt, die dazu bestimmt ist, an der Oberfläche des wiederherzustellenden Knochens außen im Verhältnis zum Knochendefekt (10; 18) anzuliegen, wobei die Stabilisierungsöse (13) keine Revaskularisationskanäle aufweist und von mindestens einem Loch (14) für den Durchgang mindestens einer Stabilisierungsschraube durchbohrt ist, und/oder dass der Keramikblock (11) von mindestens einem Durchgangsloch (25) durchbohrt ist, das von der Oberfläche, die dazu bestimmt ist, mit dem Knochen in Kontakt zu kommen, der den Knochendefekt (10; 18) begrenzt, bis zu der freien Oberfläche reicht, wenn man von der im Knochendefekt (10; 18) platzierten Position des Blocks (11) ausgeht, im Hinblick auf den Durchgang von mindestens einer Stabilisierungsschraube (26), wobei der Block (11) mindestens in dem zur freien Oberfläche benachbarten Teil keine Revaskularisationskanäle in den Regionen aufweist, die das Loch (25) umgeben.

5. Verfahren nach einem der Ansprüche 1 bis 4, **dadurch gekennzeichnet, dass** der Block (11) keine Revaskularisationskanäle in der Region seiner freien Oberfläche aufweist, wenn man von der im Knochendefekt (10; 18) platzierten Position des Blocks (11) ausgeht.

6. Verfahren nach einem der Ansprüche 1 bis 5, **dadurch gekennzeichnet, dass** die Revaskularisationskanäle einen variablen Querschnitt haben, gerade sind oder nicht, entgegengesetzt zur Oberfläche des Blocks (11) ausmünden oder nicht, die bestimmt ist, mit dem Knochendefekt (10; 18) in Kontakt zu kommen.

7. Verfahren nach einem der Ansprüche 1 bis 6, **dadurch gekennzeichnet, dass** die Kanäle, die das Revaskularisationssystem bilden, einen quadratischen Querschnitt haben, dessen Seite 250 bis 600 um mit einer Toleranz von 200 um beträgt.

8. Verfahren nach einem der Ansprüche 1 bis 7, **dadurch gekennzeichnet, dass** die Kanäle, die das Revaskularisationssystem bilden, einen größeren Querschnitt in der Region des Blocks (11) haben, die bestimmt ist, mit dem Knochen im Kontakt zu sein, der den Knochendefekt (10; 18) begrenzt, insbesondere Kanäle mit quadratischem Querschnitt von 400 bis 600 um Seitenlänge mit einer Toleranz von 200 um sind, wobei die Kanäle im Kern des Teils Kanäle mit einem quadratischen Querschnitt mit kleinerer Seitenlänge sind, oder die Dichte der Kanäle, die das Revaskularisationssystem bilden, größer in der oder den Regionen des Blocks (11) ist, die bestimmt sind, mit dem Knochendefekt (10; 18) im Kontakt zu sein.

9. Verfahren nach einem der Ansprüche 1 bis 8, **dadurch gekennzeichnet, dass** das Keramikmaterial, das den Block (11) bildet, eine intergranulare Mikroporosität von 5 bis 30 Vol.-% hat, wobei die Mikroporen eine Abmessung von 0,1 bis 10 um haben.

10. Verfahren nach einem der Ansprüche 1 bis 9, **dadurch gekennzeichnet, dass** der angestrebte synthetische Block anhand der Schritte hergestellt wird, die bestehen aus:
- Bilden, auf einer festen Unterlage oder auf einem Teil, das sich in der Herstellung befindet, einer ersten Schicht einer photohärtbaren Zusammensetzung, die mindestens ein Keramikmaterial und ein photohärtbares Monomer umfasst;
- Härtenlassen der ersten Schicht der photohärtbaren Zusammensetzung durch Bestrahlung gemäß dem für die Schicht definierten Motiv, wodurch eine erste Stufe gebildet wird;
- Bilden, auf der ersten Stufe, einer zweiten Schicht der photohärtbaren Zusammensetzung;
- Härtenlassen der zweiten Schicht der photohärtbaren Zusammensetzung durch Bestrahlung gemäß dem für die Schicht definierten Motiv, wodurch eine zweite Stufe gebildet wird;
- optional Wiederholen der Schritte, um ein Teil im Rohzustand zu erhalten;
- Reinigen des Teils im Rohzustand, um die nicht gehärtete Zusammensetzung zu entfernen;
- optional Entbindern des gereinigten Teils im Rohzustand;
- Sintern des gereinigten und optional entbinderten Teils im Rohzustand, um das fertige Teil zu erhalten.

## Claims

1. A method for manufacturing a synthetic block intended for filling in a bone defect (10;18) at the surface of a bone, in particular a bone defect occurring within the bone of a mandible or a maxilla, **characterized in that** it comprises the following steps:
- acquiring a three-dimensional image of a patient's bone having the bone defect (10;18) to be filled in;
- designing, by computer-aided design, a computing model of the synthetic block which shape corresponds to the bone defect (10;18), which has the revascularization channels and which sizes are slightly larger than said bone defect (10;18) so as to take into account the shrinkage of the ceramic when manufacturing the synthetic block;
- changing this computing model of the synthetic block, by computer-aided design, to ensure the stabilization of said synthetic block within said bone defect (10;18); and
- manufacturing the desired synthetic block, made of ceramic material, by stereolithography, an ordered three-dimensional network of channels communicating with one another being at least partially formed within said block (11) for allowing through the fluids and cells that enable revascularization for cell growth once said block (11) is placed within the bone defect (10;18), said channels opening onto each surface of the bone defect (10;18) in contact with said block (11) once it is placed within the bone defect (10;18).

2. The method according to claim 1, **characterized in that** the ceramic material is a ceramic material which is at least partially resorbable, or a non-resorbable ceramic material.

3. The method according to one of claims 1 and 2, **characterized in that** the ceramic material is selected among β-tricalcium phosphate (β-TCP), hydroxyapatite and mixtures thereof in any proportion, being particularly composed of, for 100 wt.%, 40-100 wt.% of hydroxyapatite and 0-60 wt.% of β-TCP.

4. The method according to one of claims 1 to 3, **characterized in that** the ceramic block (11) externally has, integral therewith, at least one stabilization eyelet (13) intended to abut against the surface of the bone to be restored, outside said bone defect (10;18), said stabilization eyelet (13) being not provided with revascularization channels and being pierced with at least one hole (14) for passing at least one stabilization screw, and/or said ceramic bloc (11) is pierced with at least one through hole (25), from the surface intended to come into contact with the bone delimiting said bone defect (10;18) to the free surface if one considers the position of the block (11) placed within the bone defect (10;18), for passing at least one stabilization screw (26), said block (11) being not provided with revascularization channels within the regions surrounding said hole (25) at least in the neighbouring part of said free surface.

5. The method according to one of claims 1 to 4, **characterized in that** the block (11) is not provided with revascularization channels within the region of its free surface if one considers the position of the block (11) placed within the bone defect (10;18).

6. The method according to one of claims 1 to 5, **characterized in that** the revascularization channels have a variable section, are rectilinear or not, and open or not at the opposite side of the surface of the block (11) intended to come into contact with the bone defect (10;18).

7. The method according to one of claims 1 to 6, **characterized in that** the channels forming the revascularization system have a square section which side is of 250-600 µm with a 200 µm tolerance.

8. The method according to one of claims 1 to 7, **characterized in that** the channels forming the revascularization system have a greater section within the region of the block (11) intended to contact the bone delimiting the bone defect (10;18), namely being square-section channels with a 400-600 µm side with a 200 µm tolerance, the core channels of the part being square-section channels with a smaller side, or the density of the channels forming the revascularization system is higher within the one or more regions of the block (11) intended to contact the bone defect (10;18).

9. The method according to one of claims 1 to 8, **characterized in that** the ceramic material constituting the block (11) has an intergranular microporosity of 5-30 % in volume, the micropores having a size of 0.1-10 µm.

10. The method according to one of claims 1 to 9, **characterized in that** the desired synthetic block is manufactured with the steps consisting in:
- forming, on a rigid support or on a part being manufactured, a first thermosetting composition layer comprising at least one ceramic material and a photocurable monomer;
- curing the first photocurable composition layer, by irradiation according to the pattern defined for said layer, forming a first stage;
- forming, on the first stage, a second photocurable composition layer;
- curing the second photocurable composition layer, by irradiation according to the pattern defined for said layer, forming a second stage;
- optionally repeating said steps for providing a green part;
- cleaning the green part for removing the non-cured composition;
- optionally debinding the cleaned green part;
- sintering the optionally debinded and cleaned green part, for providing the finished part.
